# EUROPÄISCHE PATENTSCHRIFT

(11) **EP 3 648 866 B1**
(45) Veröffentlichungstag und Bekanntmachung des Hinweises auf die Patenterteilung: **21.04.2021**
(21) Anmeldenummer: 18742952.7
(22) Anmeldetag: 06.07.2018
(51) Int. Cl.: B01D 53/00, F25J 3/06, C01B 3/00, C07C 7/09

(54) **VERFAHREN UND ANLAGE ZUR TRENNTECHNISCHEN BEARBEITUNG EINES AUSGANGSGEMISCHS**
METHOD AND SYSTEM FOR PROCESSING A FEED MIXTURE IN A SEPARATION PROCESS
PROCÉDÉ ET INSTALLATION DE TRAITEMENT DE SÉPARATION D'UN MÉLANGE DE DÉPART

(30) Priorität: 06.07.2017 EP 17180033
(43) Veröffentlichungstag der Anmeldung: 13.05.2020
(73) Patentinhaber: Linde GmbH, 82049 Pullach (DE)
(72) Erfinder: PHAM DUC, Tuat, 82377 Penzberg (DE); HÖFEL, Torben, 81543 München (DE)
(74) Vertreter: Frommberger, Moritz
(86) Internationale Anmeldenummer: PCT/EP2018/068407
(87) Internationale Veröffentlichungsnummer: WO 2019/008151

(56) Entgegenhaltungen:
- EP-A1- 3 136 028
- WO-A1-2015/104153
- WO-A1-2017/001514

## Beschreibung

Die Erfindung betrifft ein Verfahren zur trenntechnischen Bearbeitung eines überwiegend Wasserstoff, Methan und Kohlenwasserstoffe mit zwei oder zwei und mehr Kohlenstoffatomen enthaltenden Ausgangsgemischs und eine entsprechende Anlage gemäß den Oberbegriffen der unabhängigen Patentansprüche.

### Stand der Technik

Verfahren und Anlagen zum Dampfspalten von Kohlenwasserstoffen sind beispielsweise im Artikel "Ethylene" in Ullmann's Encyclopedia of Industrial Chemistry, Onlineausgabe, 15. April 2007, DOI 10.1002/14356007.a10_045.pub2, beschrieben. Das Dampfspalten (engl. Steam Cracking) wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Durch Dampfspalten wird dabei ein Stoffgemisch erhalten, das zunächst als Rohgas bezeichnet wird. Dieses wird mehreren Aufbereitungsschritten, beispielsweise einer Rohgaswäsche, einer Rohgasverdichtung und einer sogenannten Primärfraktionierung unterworfen. Das auf diese Weise aufbereitete Rohgas wird anschließend einer Trennung zugeführt, die zur Gewinnung von Komponenten oder Komponentengruppen des Rohgases dient. Es kann auch vorgesehen sein, bestimmte Komponenten des Rohgases vor oder innerhalb einer entsprechenden Trennung, beispielsweise durch Hydrieren oder andere Verfahren, umzusetzen.

Eine typische Trennung umfasst dabei mehrere Trennschritte, bei denen jeweils Komponentengruppen gewonnen werden. Beispielsweise sind hierbei "Demethanizer First"- (bzw. "Frontend Demethanizer"-), "Deethanizer First"- (bzw. "Frontend Deethanizer"-) oder auch "Depropanizer First"- (bzw. "Frontend Depropanizer"-) Verfahren bekannt. Zu Details sei auf die Fachliteratur, beispielsweise den erwähnten Artikel "Ethylene" in Ullmann's Encyclopedia of Industrial Chemistry, verwiesen.

Die WO 2017/001514 A1 betrifft ein Verfahren zur Gewinnung von Wasserstoff aus einem gasförmigen Einsatzgemisch, das an Wasserstoff, Methan und Kohlenwasserstoffen mit zwei Kohlenstoffatomen angereichert ist. Das Einsatzgemisch wird auf einem ersten Druckniveau von einem ersten Temperaturniveau auf ein zweites Temperaturniveau abgekühlt, so dass ein oder mehrerer Kondensate von dem Einsatzgemisch abgetrennt werden und ein Restgas verbleibt. Das Restgas wird weiter auf ein drittes Temperaturniveau abgekühlt und auf dem ersten Druckniveau unter Erhalt eines an Wasserstoff und Methan angereicherten Kopfgases und einer Sumpfflüssigkeit einer Gegenstromabsorption unterworfen. Das Kopfgas wird erwärmt und auf dem ersten Druckniveau einer Druckwechseladsorption unterworfen, wodurch ein an Wasserstoff angereicherter Produktstrom erhalten wird, der einen geringen Gehalt an Methan aufweist oder frei von Methan ist, und das oder die Kondensate und die Sumpfflüssigkeit werden von dem ersten Druckniveau auf ein zweites Druckniveau entspannt und auf dem zweiten Druckniveau einem Niederdruckdemethanizer zugeführt. Entsprechend der Erfindung wird die Gegenstromabsorption unter Verwendung von Fluid durchgeführt, das aus dem Niederdruckdemethanizer auf dem zweiten Druckniveau in gasförmigem Zustand abgezogen, auf das erste Druckniveau verdichtet, und auf das dritte Temperaturniveau abgekühlt wird. Diese Druckschrift bezieh sich außerdem auf ein entsprechendes System.

Die vorliegende Erfindung betrifft dabei insbesondere Trennungen, in denen ein überwiegend Wasserstoff, Methan und Kohlenwasserstoffe mit zwei Kohlenstoffatomen enthaltendes Stoffgemisch gebildet wird, aus welchem die Kohlenwasserstoffe mit zwei Kohlenstoffatomen unter Abtrennung von Methan und leichter siedenden Verbindungen wie Wasserstoff zunächst in einer gemeinsamen Fraktion gewonnen werden. Ein derartiges, überwiegend Wasserstoff, Methan und Kohlenwasserstoffe mit zwei Kohlenstoffatomen enthaltendes bzw. an diesen Komponenten reiches Stoffgemisch wird nachfolgend auch als "Ausgangsgemisch" bezeichnet. Grundsätzlich kann die vorliegende Erfindung jedoch auch zur trenntechnischen Bearbeitung von entsprechenden Ausgangsgemischen eingesetzt werden, die überwiegend Wasserstoff, Methan und Kohlenwasserstoffe mit zwei und mehr Kohlenstoffatomen enthalten. In ersterem Fall wird das Ausgangsgemisch in im Rahmen der vorliegenden Erfindung in einem Demethanizer eines "Deethanizer First"-Verfahrens, im letzteren Fall in einem Demethanizer eines "Demethanizer First"-Verfahrens behandelt.

Wie auch nachfolgend noch erläutert, erweisen sich in bestimmten Fällen aus dem Stand der Technik bekannte Verfahren zur trenntechnischen Bearbeitung entsprechender Ausgangsgemische als nicht zufriedenstellend, insbesondere wenn dem Dampfspaltverfahren Kohlenwasserstoffe in einem bestimmten Spektrum als Einsätze unterworfen werden.

Die vorliegende Erfindung stellt sich daher die Aufgabe, die trenntechnische Bearbeitung entsprechender Einsatzgemische zu verbessern und insbesondere energetisch effektiver durchzuführen.

### Offenbarung der Erfindung

Diese Aufgabe wird durch ein Verfahren zur trenntechnischen Bearbeitung eines überwiegend Wasserstoff, Methan und Kohlenwasserstoffe mit zwei oder zwei und mehr Kohlenstoffatomen enthaltenden Ausgangsgemischs und eine entsprechende Anlage mit den Merkmalen der unabhängigen Patentansprüche gelöst. Ausgestaltungen sind jeweils Gegenstand der abhängigen Patentansprüche und der nachfolgenden Beschreibung.

Gängige Verfahren zur Trennung von Produktströmen aus Verfahren zur Herstellung von Kohlenwasserstoffen wie des eingangs erwähnten Spaltgases umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen sowie ggf. Wasserstoff und Methan. Entsprechende Fraktionen können auch als Kältemittel eingesetzt werden, so beispielsweise die C2- oder C3-Fraktionen. Die durch entsprechende C2- oder C3-Kältemittel bereitstellbaren Temperaturniveaus werden landläufig auch als "C2-Kälte" oder "C3-Kälte" bezeichnet. Diese Kältemittel werden in Kältekreisläufen geführt, wo sie zuerst auf ein bestimmtes Enddruckniveau verdichtet und ausgehend von diesem anschließend auf unterschiedliche Druckniveaus zur Kälteerzeugung bei entsprechenden Temperaturniveaus entspannt werden.

Flüssige und gasförmige Ströme bzw. flüssige und gasförmige Gemische können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Flüssige und gasförmige Ströme bzw. flüssige und gasförmige Gemische können im hier verwendeten Sprachgebrauch ferner angereichert oder abgereichert an einer oder mehreren Komponenten sein, wobei sich diese Begriffe auf einen entsprechenden Gehalt in einem Ursprungsgemisch beziehen, aus dem der flüssige oder gasförmige Stoffstrom bzw. das flüssige oder gasförmige Gemisch jeweils erhalten wurde. Der flüssige oder gasförmige Stoffstrom bzw. das flüssige oder gasförmige Gemisch ist dabei "angereichert", wenn dieser bzw. dieses zumindest den 1,1-fachen, 1,5-fachen, 2-fachen, 5-fachen, 10-fachen, 100-fachen oder 1.000-fachen Gehalt, "abgereichert", wenn er bzw. es höchstens den 0,9-fachen, 0,5-fachen, 0,1-fachen, 0,01-fachen oder 0,001-fachen Gehalt einer entsprechenden Komponente, bezogen auf das Ursprungsgemisch, enthält. Ein "überwiegend" eine oder mehrere Komponenten enthaltender Stoffstrom bzw. ein entsprechendes Gemisch enthält diese eine oder mehreren Komponenten zumindest zu 80% oder ist reich an dieser bzw. diesen im soeben erläuterten Sinn.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Zur Auslegung und spezifischen Ausgestaltung von Rektifikationskolonnen und Absorptionskolonnen, wie sie auch im Rahmen der vorliegenden Anmeldung eingesetzt werden können, sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

### Vorteile der Erfindung

Zur trenntechnischen Behandlung der Ausgangsgemische, wie sie im Rahmen der vorliegenden Erfindung bearbeitet werden, kommen herkömmlicherweise ausschließlich oder überwiegend destillative Verfahren zum Einsatz, die je nach den beim Dampfspalten umgesetzten Verbindungen und der entsprechenden Zusammensetzung der Ausgangsgemische unterschiedlich ausgestaltet sein können. Diesen Verfahren ist gemeinsam, dass hierbei Temperaturen von -100 °C und weniger zum Einsatz kommen. Zur Erzeugung entsprechend tiefer Temperaturen können unterschiedliche Verfahren zum Einsatz kommen. Beispielsweise ist die Verwendung von kaskadierten Kältekreisläufen bekannt. Beispielsweise kann hierbei die Verwendung von C3-Kältemittel(n) (Propan und/oder Propylen, bis ca. -40 °C) gefolgt von C2-Kältemittel(n) (Ethylen, bis ca. -100 °C) und anschließend Methan-(CI-) oder Gemischkältemittel(n) (bis ca. -160 °C) erfolgen. E ntsprechende Ausgangsgemische können außerdem unter Verwendung von kalten Fraktionen oder Produktströmen, die aus den Ausgangsgemischen selbst gebildet werden, abgekühlt werden. Letztere können in bekannten Verfahren in geeigneter Weise zur Gewinnung von Kälte entspannt und anschließend wieder in einem Rohgasverdichter verdichtet werden, so dass "offene" Kältemittelkreisläufe gebildet werden können. Auch eine Kombination der genannten Maßnahmen ist möglich.

In einigen bekannten Verfahren wird eine Methan und Wasserstoff enthaltende Fraktion, die aus einem entsprechenden Einsatzgemisch abgetrennt wird, als Kältemittel eingesetzt und auf niedrigem Druckniveau als sogenanntes Tailgas an die Anlagengrenze abgegeben. Dies ist jedoch nicht immer erwünscht, insbesondere bei Einsatz von Dampfspaltverfahren, in denen flüssige Einsätze wie Naphtha oder gemischte Einsätze, die jedoch entsprechende flüssige Einsätze umfassen, zugeführt werden. Hier ist es häufig erwünscht, eine separate wasserstoffreiche Fraktion auf hohem Druckniveau und eine methanreiche Fraktion auf niedrigem Druckniveau zu erzeugen. Dabei kann die methanreiche Fraktion als Tailgas bzw. Brenngas energetisch verwendet werden, wofür typischerweise nur ein geringes Druckniveau erforderlich ist, und die separate wasserstoffreiche Fraktion kann stofflich verwendet werden, beispielsweise für Hydrierungen in der Anlage und/oder als wertschöpfendes Anlagenprodukt, wofür typischerweise ein hohes Druckniveau erforderlich ist. Typischerweise kann dieses Ziel bei Verwendung entsprechender Einsätze durch den Einsatz von konventionellen C3- und C2- Kältekreisläufen (als Kaskade) sowie unter Zuhilfenahme einer gebildeten kalten, flüssigen Methanproduktfraktion als Kältemittel, erreicht werden. Weitere Maßnahmen wie der Einsatz von C1- bzw. Gemischkältemitteln, Expandern oder Recycleströmen zur Rohgasverdichtung sind nicht erforderlich.

Die separate Gewinnung einer wasserstoffreichen Fraktion auf hohem Druckniveau wird jedoch umso schwerer, je höher der Wasserstoffanteil im Verhältnis zum Methananteil in einem entsprechenden Ausgangsgemisch, das zur trenntechnischen Bearbeitung geführt wird, ist. Dieses Verhältnis von Wasserstoff zu Methan im Ausgangsgemisch hängt wiederum von den Einsätzen zur Dampfspaltung ab. Bei überwiegend flüssigen Einsätzen liegt das Verhältnis von Wasserstoff zu Methan im Ausgangsgemisch zur trenntechnischen Bearbeitung typischerweise unter 1 mol/mol. Erhöhte Wasserstoffanteile ergeben sich in sogenannten "Mixed Feed"-Dampfspaltverfahren, also in Dampfspaltverfahren, bei denen sowohl gasförmige Einsätze wie Ethan als auch die zuvor erläuterten flüssigen Einsätze unterworfen werden. Abhängig von der Mischung der Einsatzstoffe liegt in derartigen Dampfspaltverfahren das Verhältnis von Wasserstoff zu Methan im Ausgangsgemisch zur trenntechnischen Bearbeitung beispielsweise zwischen 1 mol/mol und 2 mol/mol, insbesondere zwischen 1,2 mol/mol und 1,8 mol/mol.

In derartigen Fällen steht die Menge der gebildeten flüssigen, kalten Methanproduktfraktion in zu geringer Menge als Kältemittel zur Verfügung, so dass auf andere Möglichkeiten zurückgegriffen werden muss. Der dabei grundsätzlich denkbare Einsatz von geschlossenen C1- bzw. Gemischkältekreisläufen ist jedoch aufwendig und teuer, Recycleströme zur Rohgasverdichtung sind energieineffizient. Würde beispielsweise ein Expander eingesetzt, ginge dies auf Kosten der gewinnbaren Wasserstoffproduktmenge.

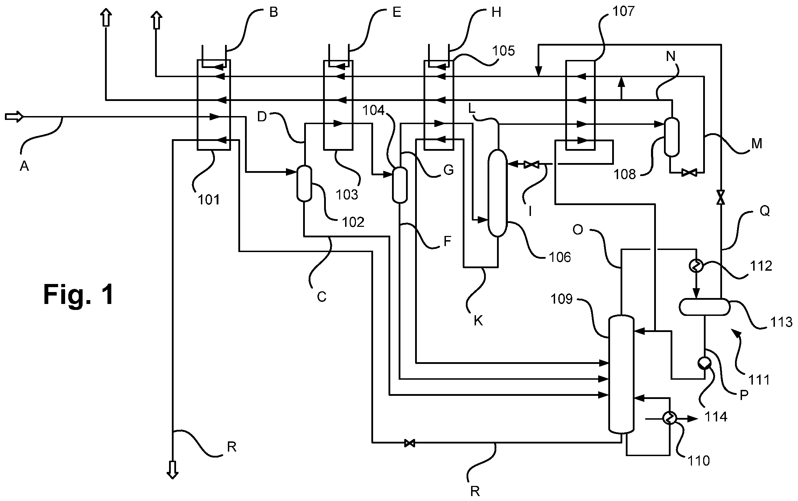

Die vorliegende Erfindung nutzt nun den Umstand, dass in herkömmlichen Verfahren der erläuterten Art neben der flüssigen Methanproduktfraktion typischerweise auch eine gasförmige Methanproduktfraktion erzeugt wird. Hierzu sei beispielsweise auf die beigefügte Figur 1 verwiesen. In dem dort veranschaulichten Verfahren werden, wie auch unten nochmals erläutert, zunächst eine flüssige, methanreiche Fraktion in Form der Sumpfflüssigkeit des Abscheidebehälters 108, die anschließend in Form des Stoffstroms M bereitgestellt wird, und eine gasförmige methanreiche Fraktion in Form des Kopfgases des Abscheidebehälters 113, die anschließend in Form des Stoffstroms Q bereitgestellt wird, gebildet.

Im Rahmen der vorliegenden Erfindung wird nun die erwähnte gasförmige Methanfraktion zumindest teilweise in unveränderter Zusammensetzung auf einen derart hohen Druck gebracht, dass wesentliche Teile dieser Methanfraktion unter Verwendung von C2-Kälte kondensiert werden können. Entsprechend gebildetes flüssiges Methan steht dann als zusätzliches Kältemittel zur Verfügung und das Ziel der Erzeugung einer separaten wasserstoffreichen Fraktion auf hohem Druckniveau kann erreicht werden. Hierzu ist nur ein geringes Druckverhältnis von unter 1,4 bzw. 1,6 erforderlich. Daher kann ein vergleichsweise kleiner, leistungsarmer Apparat (in der Größenordnung von 1 bis 5 % der Leistung des Rohgasverdichters) zum Einsatz kommen. Dies ist möglich, weil die gasförmige Methanfraktion leicht unter dem Niveau der C2-Kälte (ca. -97 °C) die verwendete Rektifikat ionskolonne verlässt. Diese gasförmige Methanfraktion muss daher nur noch in vergleichsweise geringem Umfang weiter verdichtet werden (ca. auf 35 bis 45 bar), um diese auf dem genannten Temperaturniveau unter Verwendung von C2-Kälte dennoch zu einem großen Teil kondensieren zu können. Die Kondensation erfolgt, damit das gewonnene Kondensat oder ein hieraus gebildeter Strom anschließend bei einemem Temperaturniveau unterhalb der C2-Kälte in einem Wärmetauscher genutzt werden kann. Hierfür ist es erforderlich, dass das Kondensat zuvor entspannt wird, beispielsweise auf 7 bar. Nach dieser Entspannung kann die entsprechende gewonnene Kälte auf dem tieferen Temperaturniveau genutzt werden. Das erfindungsgemäße Verfahren ermöglicht daher die Gewinnung einer hohen Menge einer wasserstoffreichen Fraktion auf hohem Druckniveau unter geringem zusätzlichem Aufwand.

Es sei an dieser Stelle vorsorglich erwähnt, dass der beispielsweise zu Figur 2 der WO 2015/104153 A1 erläuterte Stand der Technik im Gegensatz zur vorliegenden Erfindung den Fall betrifft, dass eine flüssige Methanfraktion mittels einer Pumpe gefördert wird. Ein gasförmiger Methanstrom wird hier nicht verdichtet, sondern lediglich als gasförmiges Kältemittel auf einem höheren Temperaturniveau als im Rahmen der vorliegenden Erfindung genutzt. Die geförderte Methanfraktion wird anschließend als Rücklauf auf einen C2-Absorber verwendet.

Die in Figur 2 der WO 2015/104153 A1 gezeigten Wärmetauscher 1 bis 3 sind durch C2-Kälte bilanziert, der Wärmetauscher 4 wird nur mit Produktströmen gekühlt. Wenn nicht genug flüssiges Methan zur Verfügung steht (d.h. ein hohes Verhältnis von Wasserstoff zu Methan vorliegt) wird im Gegensatz zur WO 2015/104153 A1 erfindungsgemäß der dort gezeigte Strom I auf einen derart hohen Druck gebracht, dass dieser bereits im Wärmetauscher gegen C2-Kälte überwiegend kondensiert werden kann, damit dieser anschließend als Kältemittel im Wärmetauscher 4 nutzbar ist. Es handelt sich also bei den im Rahmen der vorliegenden Erfindung um eine konventionelle Kälteanlage (Verdichtung, Kondensation, Entspannung, Verdampfung). Jedoch wird kein "offener Kältekreislauf", sondern eine "offene Kälteerzeugung" eingesetzt, da jedes Methanmolekül aus dem Strom I nur genau einmal durch den Verdichter geführt und anschließend auf den niedrigen Tailgasdruck entspannt wird.

Die vorliegende Erfindung geht von einem Verfahren zur trenntechnischen Bearbeitung eines Ausgangsgemischs, das überwiegend Wasserstoff, Methan und Kohlenwasserstoffe mit zwei oder zwei und mehr Kohlenstoffatomen enthält aus, wobei zumindest ein Teil des Ausgangsgemischs unter Bildung eines oder mehrerer Kondensate unter Verwendung eines oder mehrerer Wärmetauscher abgekühlt und zumindest ein Teil des oder der Kondensate unter Bildung einer gasförmigen methanreichen Fraktion einer Rektifikation unterworfen wird. Typischerweise erfolgt im Rahmen der vorliegenden Erfindung dabei, wie auch nachfolgend noch erläutert, eine stufenweise Abkühlung unter jeweiliger Abscheidung mehrerer Kondensate und deren Einspeisung in eine Rektifikationskolonne.

Im Rahmen des erfindungsgemäßen Verfahrens wird die gasförmige methanreiche Fraktion insbesondere auf einem Temperaturniveau von -95 bis -100 °C, vorzugsweise -96 bis -98 °C, gebildet. Dieses Tempe raturniveau liegt nahe jenem der C2-Kälte, so dass eine vergleichsweise geringfügige anschließende Verdichtung ausreicht um, wie erwähnt, die gasförmige Fraktion anschließend zumindest teilweise kondensieren zu können.

Es sei explizit betont, dass im Rahmen der vorliegenden Anmeldung insbesondere statt mehrerer getrennter Wärmetauscher auch baulich integrierte Wärmetauscherstufen in einem gemeinsamen Wärmetauscher verwendet werden können, die beispielsweise in Form mehrerer fluidisch miteinander verbundener Wärmetauscherabschnitte bzw. Wärmetauscherblöcke ausgebildet sein können. Auch in einem derartigen Wärmetauscher ist eine stufenweise Abkühlung und eine Bildung mehrerer Kondensate möglich. Wenngleich nachfolgend nicht im Detail erläutert, kann die Abkühlung aber auch grundsätzlich unter Verwendung nur einer Abkühlstufe und der Bildung nur eines Kondensats durchgeführt werden.

Grundsätzlich erfolgt die erläuterte Abkühlung im Rahmen der vorliegenden Erfindung unter Druck- und Temperaturbedingungen (siehe hierzu auch unten), mittels derer gewährleistet werden kann, dass nach der Abkühlung und der Bildung des oder der Kondensate ein gasförmig verbleibender Rest kaum oder keine Kohlenwasserstoffe mit zwei und ggf. mehr Kohlenwasserstoffen mehr enthält. Auf diese Weise wird gewährleistet, dass keine wertvollen Produkte bzw. in die Dampfspaltung rezyklierbaren Verbindungen in dem gasförmig verbleibenden Rest mehr enthalten sind und auf diese Weise verloren gehen bzw. anderenfalls nur auf sehr aufwendige Weise zurückgewonnen werden können. Insbesondere erfolgt eine entsprechende Abkühlung und Bildung des oder der Kondensate derart, dass in einem nach der Abscheidung des oder der Kondensate gasförmig verbleibender Rest nicht mehr als 1 Molprozent, vorzugsweise nicht mehr als 0,1 Molprozent, besonders vorzugweise nicht mehr als 0,01 Molprozent, an Kohlenwasserstoffen mit zwei und ggf. mehr Kohlenstoffatomen enthält.

Die Bildung des oder der Kondensate erfolgt damit im Hinblick auf eine weitgehende Abreicherung an Kohlenwasserstoffen mit zwei und ggf. mehr Kohlenstoffatomen. Hiermit geht einher, dass gewisse Mengen an Methan und Wasserstoff zusammen mit den Kohlenwasserstoffen mit zwei und ggf. mehr Kohlenstoffatomen ebenfalls aus einem entsprechenden Ausgangsgemisch in das oder die Kondensate abgeschieden werden. Die bereits erwähnte Rektifikation dient nun vornehmlich dazu, das Methan und den Wasserstoff von Kohlenwasserstoffen mit zwei und ggf. mehr Kohlenstoffatomen abzutrennen. Wie erwähnt, kann dies insbesondere bei der Verwendung von gemischten Einsätzen in einem Dampfspaltverfahren problematisch sein, da bei zunehmenden Verhältnissen von Wasserstoff zu Methan in den Kondensaten zur Rektifikation ein immer höherer Kolonnendruck und/oder eine immer geringere Temperatur im Kopfkondensator der Destillation erforderlich ist.

Insbesondere kann das erfindungsgemäße Verfahren daher zum Einsatz kommen, wenn im Ausgangsgemisch zur trenntechnischen Bearbeitung das Verhältnis von Wasserstoff zu Methan beispielsweise zwischen 1 mol/mol und 2 mol/mol, insbesondere zwischen 1,2 mol/mol und 1,8 mol/mol, liegt. Ein derartiges Ausgangsgemisch kann insbesondere aus einem Spaltgas bzw. Rohgas eines Dampfspaltverfahrens gewonnen werden, dem die zuvor erläuterten gemischten Einsätze zugeführt werden. Für herkömmliche Verhältnisse von Wasserstoff zu Methan unter 1 mol/mol können herkömmliche Trennverfahren zum Einsatz kommen, die typischerweise aus Anlagen zur Dampfspaltung von flüssigen Einsätzen bekannt sind. Für Verhältnisse von Wasserstoff zu Methan über 2 mol/mol können herkömmliche Trennverfahren zum Einsatz kommen, die typischwerise aus Anlagen zur Dampfspaltung von Ethan bekannt sind, beispielsweise unter Ausnutzung der Kälte in der wasserstoffreichen Fraktion, die dann nicht mehr als Hochdruckfraktion an die Anlagengrenze gefahren werden kann. Die vorliegende Erfindung betrifft hingegen insbesondere den speziellen Fall der Verwendung gemischter Einsätze beim Dampfspalten.

Erfindungsgemäß ist vorgesehen, dass die gasförmige methanreiche Fraktion zumindest zum Teil zur Bildung eines ersten Fluidstroms verwendet wird, der in unveränderter Zusammensetzung gegenüber der gasförmigen methanreichen Fraktion auf ein Verflüssigungsdruckniveau von 35 bis 45 bar, vorzugsweise von 35 bis 40 bar, besonders vorzugsweise 35 bis 38 bar, verdichtet, durch Abkühlen zumindest teilweise verflüssigt, und, in noch immer unveränderter Zusammensetzung gegenüber der gasförmigen methanreichen Fraktion, auf ein geringeres Druckniveau, hier auch als "Abgabedruckniveau" bezeichnet, entspannt wird. Ist hier davon die Rede, dass der erste Fluidstrom, d.h. der Teil der methanreichen Fraktion, der verdichtet, zumindest teilweise verflüssigt und anschließend entspannt wird, diesen Schritten "in gegenüber der gasförmigen methanreichen Fraktion unveränderter Zusammensetzung" unterworfen wird, soll dies ausdrücken, dass sich die relativen Anteile von Komponenten, sei es dass diese in flüssiger, gasförmiger oder gemischtphasiger Form vorliegen, in diesem Fluidstrom insgesamt nicht ändern. Der erste Fluidstrom wird insbesondere nicht unter Veränderung seiner Zusammensetzung mit anderen Fluidströmen vermischt und er wird keiner Trennung oder einem Schritt unterworfen, in dem er in eine Gas- und eine Flüssigphase mit unterschiedlichen Zusammensetzungen getrennt wird, wobei nur eine dieser Phasen weiter verwendet wird. Insbesondere wird dieser erste Fluidstrom nicht in einer Absorptionskolonne eingesetzt und darin unter Bildung weiterer Fluidströme unterschiedlicher Zusammensetzung einer Abreicherung oder Anreicherung an Komponenten unterworfen. Insbesondere werden die Verfahrensschritte der Verdichtung, Verflüssigung und Entspannung direkt nacheinander durchgeführt, insbesondere ohne zwischengeschaltete weitere Schritte (abgesehen von beispielsweise einer Erwärmung und Abkühlung oder einem Transfer durch Leitungen). Dies schließt jedoch nicht aus, dass der erste Fluidstrom in Form eines Zweiphasenstroms mit einer flüssigen und einer gasförmigen Phase vorliegen kann, wenn er nicht vollständig verflüssigt wird. Jedoch ändert sich die Zusammensetzung des ersten Fluidstroms insgesamt, die die Summe der Zusammensetzungen in der flüssigen und der gasförmigen Phase ist, auch in diesem Fall nicht, selbst wenn die individuellen Zusammensetzungen der beiden Phasen unterschiedlich sein sollte.

Der erste Fluidstrom, oder ein zweiter Fluidstrom, der unter Verwendung des ersten Fluidstroms gebildet wird, wird anschließend an die Entspannung des ersten Fluidstroms auf das Abgabedruckniveau, bei welcher er noch immer dieselbe Zusammensetzung aufweist wie die gasförmige, methanreiche Fraktion, in dem oder zumindest einem der Wärmetauscher erwärmt. Auf diese Weise kann ungeachtet der geringeren Methanmengen eine Bereitstellung einer wasserstoffreichen, gasförmigen Produktfraktion auf einem ausreichend hohen Druckniveau ermöglicht werden, wie zuvor erläutert. Dies wird insbesondere dadurch erreicht, dass bei dem beim Abkühlen des Ausgangsgemischs oder von dessen Teil in dem oder den Wärmetauschern Wärme auf die verdichtete und zumindest teilweise verflüssigte methanreiche Fraktion oder deren Teil in dem oder den Wärmetauschern übertragen wird.

Vorzugsweise wird dabei das Abkühlen des Ausgangsgemischs oder von dessen Teil in dem oder den Wärmetauschern auf einem Abkühldruckniveau unterhalb des Verflüssigungsdruckniveaus durchgeführt. Auf diesem kann auch die wasserstoffreiche, gasförmige Produktfraktion bereitgestellt werden, die im Rahmen des erfindungsgemäßen Verfahrens vorzugsweise keiner Entspannung unterworfen werden muss. Hingegen wird die Rektifikation im Rahmen der vorliegenden Erfindung vorzugsweise auf einem Rektifikationsdruckniveau geringfügig unterhalb des Abkühldruckniveaus durchgeführt, damit die Kondensate ohne Pumpe zur Rektifikation geführt werden können. Das Abkühldruckniveau liegt dabei insbesondere bei 25 bis 40 bar, vorzugsweise bei 30 bis 38 bar, besonders vorzugsweise bei 32 bis 38 bar. Das Rektifikationsdruckniveau liegt im Rahmen der Erfindung insbesondere 0,2 bis 4 bar, vorzugsweise 1 bis 3 bar, besonders vorzugsweise 2 bis 3 bar unterhalb des Abkühldruckniveaus.

Wie nun bereits mehrfach erwähnt, kann im Rahmen der vorliegenden Erfindung insbesondere ungeachtet der geringeren Gehalte an Methan in einem entsprechenden Ausgangsgemisch eine wasserstoffreiche Produktfraktion auf einem zufriedenstellenden Druckniveau bereitgestellt werden. Ein Verfahren gemäß einer besonders bevorzugten Ausgestaltung des erfindungsgemäßen Verfahrens umfasst daher, eine bei dem Abkühlen des Ausgangsgemischs oder von dessen Teil in dem oder den Wärmetauschern gasförmig bleibende, wasserstoffreiche Fraktion ebenfalls in dem oder zumindest einem der Wärmetauscher zu erwärmen. Die wasserstoffreiche Fraktion verbleibt dabei insbesondere stromab eines Abkühlschritts auf einem Temperaturniveau von -120 bis -170 °C, vorzugsweise -140 bis -165 °C, beispielsweise ca. -160 °C, bei dem Methan zumindes t zum überwiegenden Anteil auskondensiert. Entsprechend tiefe Temperaturen lassen sich insbesondere durch Verwendung eines methanreichen Kältemittels, im Rahmen der vorliegenden Erfindung unter anderem unter Verwendung der verdichteten und zumindest teilweise verflüssigten methanreichen Fraktion, erzielen. Die gasförmig verbleibende Fraktion enthält daher insbesondere 80 bis 100 Molprozent, vorzugsweise 85 bis 95 Molprozent, beispielsweise ca. 90 Molprozent, Wasserstoff.

Um eine entsprechende wasserstoffreiche Produktfraktion bereitzustellen, ist es im Rahmen der vorliegenden Erfindung insbesondere vorgesehen, die wasserstoffreiche Fraktion unentspannt zu erwärmen. Vorzugsweise wird die Erwärmung der wasserstoffreichen Fraktion oder deren Teil im Rahmen der vorliegenden Erfindung daher auf dem Abkühldruckniveau vorgenommen.

Wie erwähnt wird im Rahmen der vorliegenden Erfindung die verdichtete und zumindest teilweise verflüssigte methanreiche Fraktion oder deren Teil (d.h. der erwähnte "erste Fluistrom") vor der Erwärmung in dem oder den Wärmetauschern auf ein Abgabedruckniveau unterhalb des Abkühldruckniveaus entspannt. Das Abgabedruckniveau kann beispielsweise bei 2 bis 10 bar, insbesondere bei 5 bis 8 bar, insbesondere bei ca. 7 bar, liegen, Auf diese Weise kann Kälte generiert und im Rahmen der vorliegenden Erfindung für das Ausgangsgemisch bzw. dessen verwendeten Anteil genutzt werden.

Weitere methanreiche Fraktionen können im Rahmen der vorliegenden Erfindung ebenfalls zum Einsatz kommen. So kann gemäß einer besonders vorteilhaften Ausführungsform des erfindungsgemäßen Verfahrens mittels der Rektifikation eine flüssige, methanreiche Fraktion gebildet werden, die zumindest zum Teil zusammen mit der verdichteten und zumindest teilweise verflüssigten methanreichen Fraktion oder deren Teil in dem oder den Wärmetauschern erwärmt wird. Auf diese Weise kann auch eine derartige Fraktion, wenngleich in geringerem Anteil vorhanden, entsprechend genutzt werden.

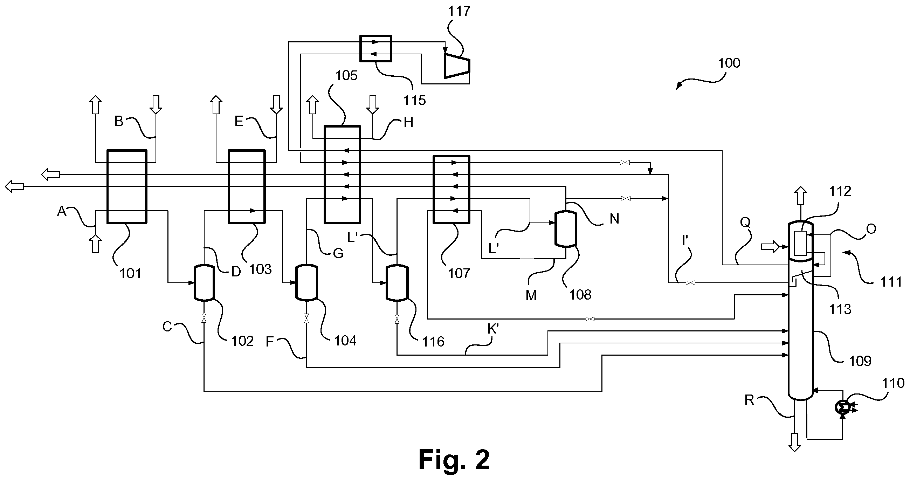

Im Detail kann ein Verfahren besonders vorteilhaft sein, wie es auch unter Bezugnahme auf die beigefügte Figur 2 erläutert ist. Hierbei erfolgt ein Abkühlen unter Verwendung eines ersten Wärmetauschers, eines zweiten Wärmetauschers, eines dritten Wärmetauschers und eines vierten Wärmetauschers, die seriell angeordnet sind und durch die das Ausgangsgemisch oder dessen verwendeter Anteil unter kontinuierlicher Abreicherung an den Kohlenwasserstoffen mit zwei und ggf. mehr Kohlenstoffatomen (sowie Methan und geringen Mengen Wasserstoff) geführt wird. In einem Verfahren gemäß einer besonders bevorzugten Ausgestaltung der vorliegenden Erfindung wird das Ausgangsgemisch oder dessen Teil also nacheinander durch den ersten, den zweiten, den dritten und den vierten Wärmetauscher geführt, wobei stromab jedes Wärmetauschers jeweils ein Kondensat abgeschieden wird. Besonders bevorzugt ist dabei, wenn die Bildung der Kondensate rein kondensativ, d.h. ohne Absorptionskolonnen wie im Stand der Technik, erfolgt.

Insbesondere kann dabei im Rahmen der vorliegenden Erfindung der erste Wärmetauscher unter Verwendung eines ethylenreichen Kältemittels (z.B. sogenanntem Hochdruckethylen) mit -50 bis -60 °C, v orzugsweise ca. -57 °C, der zweite Wärmetauscher unter Verwendung eines ethylenreichen Kältemittels (z.B. sogenanntem Mitteldruckethylen) mit -75 bis -85 °C, vorzugsweise ca. -80 °C, und der dritte Wärmetauscher unter Verwendung eines ethylenreichen Kältemittels (z.B. sogenanntem Niederdruckethylen) mit -95 bis -105 °C, vorzugsweise ca. -100 °C, betrieben werden. Entsprechende Temperaturniveaus werden dabei im Rahmen des erläuterten Abkühldruckniveaus eingesetzt.

Wie erwähnt, können im Rahmen der vorliegenden Erfindung in einem letzten Abkühlschritt Fraktionen des Ausgangsgemischs eingesetzt werden. Die vorliegende Erfindung sieht insbesondere vor, durch den vierten Wärmetauscher Fraktionen einer nach dem Abkühlen in dem dritten Wärmetauscher gasförmig verbliebenen und zuvor in dem vierten Wärmetauscher abgekühlten Fraktion zu erwärmen. Auf diese Weise lässt sich im Rahmen der vorliegenden Erfindung insbesondere ein Temperaturniveau von -120 bis -180 °C, insbesondere ca. -160 °C, erz ielen.

Im Rahmen der soeben erläuterten Ausführungsform des erfindungsgemäßen Verfahrens ist insbesondere vorgesehen, dass die gasförmige methanreiche Fraktion oder deren verwendeter Teil (d.h. der erwähnte "erste Fluistrom") nacheinander in dem dritten Wärmetauscher erwärmt, durch einen weiteren Wärmetauscher geführt, auf das Verflüssigungsdruckniveau verdichtet, durch den weiteren Wärmetauscher geführt und in dem dritten und vierten Wärmetauscher abgekühlt wird. Anschließend wird die nunmehr teilweise oder vollständig verflüssigte, zuvor gasförmige methanreiche Fraktion in dem vierten, dem dritten, dem zweiten und dem ersten Wärmetauscher erwärmt.

Die vorliegende Erfindung betrifft auch eine Anlage zur trenntechnischen Bearbeitung eines Ausgangsgemischs, das überwiegend Wasserstoff, Methan und Kohlenwasserstoffe mit zwei oder zwei und mehr Kohlenstoffatomen enthält, mit Mitteln, die dafür eingerichtet sind, zumindest ein Teil des Ausgangsgemischs unter Bildung eines oder mehrerer Kondensate unter Verwendung eines oder mehrerer Wärmetauscher abzukühlen und zumindest ein Teil des oder der Kondensate unter Bildung einer gasförmigen methanreichen Fraktion einer Rektifikation zu unterwerfen, Erfindungsgemäß sind Mittel vorgesehen, die dafür eingerichtet sind, die methanreiche Fraktion zur Bildung eines ersten Fluidstroms zu verwenden, und durch Mittel, die dafür eingerichtet sind, den ersten Fluidstrom in unveränderter Zusammensetzung gegenüber der gasförmigen methanreichen Fraktion zumindest zum Teil auf ein Verflüssigungsdruckniveau von 35 bis 45 bar, insbesondere die zuvor erwähnten Werte, zu verdichten, durch Abkühlen zumindest teilweise zu verflüssigen, und auf ein Abgabedruckniveau zu entspannen. Weitere Mittel sind dafür eingerichtet, den ersten Fluidstrom oder einen zweiten, unter Verwendung des ersten Fluidstroms gebildeten Fluistrom in dem oder zumindest einem der Wärmetauscher zu erwärmen.

Zu Merkmalen und Vorteilen einer entsprechenden Anlage, die vorteilhafterweise zur Durchführung eines Verfahrens eingerichtet ist, wie es in Ausgestaltungen zuvor erläutert wurde, sei auf die obigen Ausführungen ausdrücklich verwiesen.

Eine Ausführungsform der Erfindung wird nachfolgend unter Bezugnahme auf die beigefügten Zeichnungen gegenüber dem Stand der Technik näher erläutert.

Kurze Beschreibung der Zeichnungen
Figur 1 zeigt ein nicht erfindungsgemäßes Verfahren in Form eines schematischen Prozessflussdiagramms.
Figur 2 zeigt ein Verfahren gemäß einer Ausführungsform der Erfindung in Form eines schematischen Prozessflussdiagramms.

### Ausführliche Beschreibung der Zeichnungen

In den Figuren tragen einander entsprechende Elemente entsprechende Bezugszeichen und werden der Übersichtlichkeit halber nicht wiederholt erläutert.

In Figur 1 ist ein nicht erfindungsgemäßes Verfahren zur trenntechnischen Bearbeitung eines überwiegend Wasserstoff, Methan und Kohlenwasserstoffen mit zwei Kohlenstoffatomen enthaltenden Ausgangsgemischs in Form eines schematischen Prozessflussdiagramms zur Veranschaulichung des Hintergrunds der vorliegenden Erfindung veranschaulicht. Das Verfahren wird insbesondere zur trenntechnischen Bearbeitung eines Ausgangsgemischs eingesetzt, das aus einem Rohgas eines Dampfspaltverfahrens gebildet wird, bei dem überwiegend oder ausschließlich flüssige Einsätze, beispielsweise Naphtha, bearbeitet werden.

Es sei ausdrücklich betont, dass Figur 1 lediglich zur Veranschaulichung der Grundlagen der vorliegenden Erfindung dient und die hier dargestellte spezifische Ausgestaltung der gezeigten Apparate auch abweichend vorgenommen werden kann, ohne jedoch ein grundsätzlich anderes Trennverfahren bereitzustellen.

Das Ausgangsgemisch oder ein Teil davon wird in Form des Stoffstroms A zunächst durch einen ersten Wärmetauscher 101 geführt und unter anderem unter Verwendung eines Stoffstroms B, bei welchem es sich beispielsweise um Ethylen auf einem Temperaturniveau von ca. -57 °C handeln kann, abgek ühlt.

Der Stoffstrom A wird in einen ersten Abscheidebehälter 102 überführt, welchem ein flüssiger Stoffstrom C und ein gasförmiger Stoffstrom D entnommen werden. Der gasförmige Stoffstrom D wird durch einen zweiten Wärmetauscher 103 geführt und hier unter anderem mit einem Stoffstrom E, bei welchem es sich um Ethylen auf einem Temperaturniveau von ca. -80 °C handeln kann, weite r abgekühlt.

Der Stoffstrom D wird in einen weiteren Abscheidebehälter 104 überführt, dem ein flüssiger Stoffstrom F und ein gasförmiger Stoffstrom G entnommen werden. Der Stoffstrom G wird durch einen dritten Wärmetauscher 105 geführt und dort unter anderem mit einem Stoffstrom H, bei welchem es sich um Ethylen auf einem Temperaturniveau von ca. -100 °C handeln kann, abgekühlt.

Der Stoffstrom G wird anschließend in eine Absorptionskolonne 106 überführt, welche mit einem methanreichen flüssigen Stoffstrom I als Rücklauf betrieben wird. Aus dem Sumpf der Absorptionskolonne 106 wird ein flüssiger Stoffstrom K abgezogen und im dargestellten Beispiel in dem Wärmetauscher 105 erwärmt.

Ein gasförmiger Stoffstrom L vom Kopf der Absorptionskolonne 106 wird in einem Wärmetauscher 107 weiter abgekühlt und anschließend in einen weiteren Abscheidebehälter 108, den sogenannten Wasserstoffabscheider, überführt. Aus dem Abscheidebehälter 108 werden ein flüssiger methanreicher Stoffstrom M und ein gasförmiger, wasserstoffreicher Stoffstrom N abgezogen. Der Stoffstrom M wird auf ein niedrigeres Druckniveau entspannt, der Stoffstrom N bleibt auf dem höheren Druckniveau, auf dem er dem Abscheidebehälter 108 entnommen wird. Beide Stoffströme werden in den Wärmetauschern 107, 105, 103 und 101 erwärmt und jeweils als methanreiche bzw. wasserstoffreiche Produktfraktion bereitgestellt.

Die bereits erwähnten flüssigen Stoffströme C, F und K werden in eine Rektifikationskolonne 109 überführt, wobei ihre Einspeisung je nach Zusammensetzung und Temperatur in unterschiedlichen Höhen erfolgt. Die Rektifikationskolonne 109 wird mit einem Sumpfverdampfer 110 unter Verwendung eines typischen C3-Kältemittels betrieben. Vom Kopf der Rektifikationskolonne 109 wird ein gasförmiger Stoffstrom O abgezogen und einem insgesamt mit 111 bezeichneten Kopfkondensator zugeführt. Der Kopfkondensator 111 umfasst einen Wärmetauscher 112, der mit Ethylen auf einem Temperaturniveau von ca. -100 °C als Kältemittel betrieben werden kann. In einem dem Wärmetauscher 112 nachgeschalteten Abscheidebehälter 113 wird ein flüssiger Stoffstrom P erhalten, welcher mittels einer Pumpe 114 zu einem Teil als Rücklauf auf die Rektifikationskolonne 109 und zu einem Teil als Rücklauf auf die Absorptionskolonne 106 in Form des Stoffstroms I aufgegeben wird. Ein nicht verflüssigter Anteil des Stoffstroms O wird in Form des Stoffstroms Q aus dem Abscheidebehälter 113 abgezogen und als methanreicher Stoffstrom mit dem Stoffstrom M vereinigt.

In dem in Figur 1 veranschaulichten Verfahren werden also zunächst eine flüssige, methanreiche Fraktion in Form der Sumpfflüssigkeit des Abscheidebehälters 108, die anschließend in Form des Stoffstroms M bereitgestellt wird, und eine gasförmige methanreiche Fraktion in Form des Kopfgases des Abscheidebehälters 113, die anschließend in Form des Stoffstroms Q bereitgestellt wird, gebildet.

Durch den erläuterten Betrieb der Rektifikationskolonne 109 kann aus deren Sumpf ein flüssiger Stoffstrom R abgezogen werden, der reich an Kohlenwasserstoffen mit zwei Kohlenstoffatomen ist. Dieser wird im dargestellten Beispiel im Wärmetauscher 101 erwärmt und anschließend beispielsweise einer Trennung zur Gewinnung vom Ethan und Ethylen in einem sogenannten C2-Splitter unterworfen.

Wie bereits erläutert, wird die separate Gewinnung einer wasserstoffreichen Fraktion auf hohem Druckniveau umso schwerer, je höher der Wasserstoffanteil im Verhältnis zum Methananteil in einem entsprechenden Einsatzgemisch ist, da der Wärmetauscher 107 ausschließlich mit Produktströmen gekühlt wird und die Wärmebilanz um dem Wärmetauscher 107 umso weniger aufgeht, je höher der Wasserstoffanteil im Verhältnis zum Methananteil in einem entsprechenden Einsatzgemisch ist.

In Figur 2 ist nun ein Verfahren zur trenntechnischen Bearbeitung eines derartigen Ausgangsgemischs gemäß einer Ausführungsform der Erfindung in Form eines schematischen Prozessflussdiagramms veranschaulicht und mit 100 bezeichnet. Das Verfahren 100 wird insbesondere zur trenntechnischen Bearbeitung eines Ausgangsgemischs eingesetzt, das aus einem Rohgas eines Dampfspaltverfahrens gebildet wird, bei dem ein gemischter Einsatz, wie zuvor erläutert, bearbeitet wird.

Das Ausgangsgemisch oder ein Teil davon wird in Form eines Stoffstroms A auch hier zunächst durch einen ersten Wärmetauscher 101 geführt und unter anderem unter Verwendung eines Stoffstroms B, bei welchem es sich beispielsweise um Ethylen auf einem Temperaturniveau von ca. -57 °C handeln kann, abgekühlt.

Der Stoffstrom A wird auch hier in einen ersten Abscheidebehälter 102 überführt, welchem ein flüssiger Stoffstrom C und ein gasförmiger Stoffstrom D entnommen werden. Der gasförmige Stoffstrom D wird auch hier durch einen zweiten Wärmetauscher 103 geführt und hier unter anderem mit einem Stoffstrom E, bei welchem es sich um Ethylen auf einem Temperaturniveau von ca. -80 °C handeln kann, weiter abgekühlt.

Der Stoffstrom D wird auch hier in einen weiteren Abscheidebehälter 104 überführt, dem ein flüssiger Stoffstrom F und ein gasförmiger Stoffstrom G entnommen werden. Der Stoffstrom G wird auch hier durch einen dritten Wärmetauscher 105 geführt und dort unter anderem mit einem Stoffstrom H, bei welchem es sich um Ethylen auf einem Temperaturniveau von ca. -100 °C handeln kann, abgekühlt.

Der Stoffstrom G wird nun jedoch im Gegensatz zu dem in Figur 1 veranschaulichten Verfahren nicht in eine Absorptionskolonne 106, sondern in einen weiteren Abscheidebehälter 116 überführt, dem ein flüssiger Stoffstrom K' und ein gasförmiger Stoffstrom L' entnommen werden. Der Stoffstrom L' wird in einem Wärmetauscher 107 weiter abgekühlt und anschließend in einen weiteren Abscheidebehälter 108, der auch hier einen Wasserstoffabscheider darstellt, überführt.

Aus dem Abscheidebehälter 108 werden auch hier ein flüssiger methanreicher Stoffstrom M und ein gasförmiger, wasserstoffreicher Stoffstrom N abgezogen. Der Stoffstrom M, dessen Menge durch ein nicht gesondert bezeichnetes Ventil begrenzt wird, wird in dem Wärmetauscher 107 erwärmt. Im dargestellten Beispiel wird nur der Stoffstrom N auf dem Abkühldruckniveau nacheinander in den Wärmetauschern 107, 105, 103 und 101 erwärmt und als wasserstoffreiche Produktfraktion bereitgestellt.

Die bereits erwähnten flüssigen Stoffströme C, F und K' sowie auch der Stoffstrom M werden unter Begrenzung nicht gesondert bezeichneter Ventile in eine Rektifikationskolonne 109 überführt, wobei ihre Einspeisung je nach Zusammensetzung und Temperatur in unterschiedlichen Höhen erfolgt. Die Rektifikationskolonne 109 wird auch hier mit einem Sumpfverdampfer 110 unter Verwendung eines typischen C3-Kältemittels betrieben. Vom Kopf der Rektifikationskolonne 109 wird wie zuvor ein gasförmiger Stoffstrom O abgezogen und einem insgesamt mit 111 bezeichneten Kopfkondensator zugeführt. Der Kopfkondensator 111 ist hier jedoch in die Rektifikationskolonne 109 integriert. Er umfasst einen Wärmetauscher 112, der mit Ethylen auf einem Temperaturniveau von ca. -100 °C als Kältemittel betrieben werden kann. In einem dem Wärmetauscher 112 nachgeschalteten, hier jedoch ebenfalls in die Rektifikationskolonne 109 integrierten Abscheidebehälter 113 fällt eine flüssige Fraktion an, welche hier ohne Pumpe sondern über einen Überlauf als Rücklauf auf die Rektifikationskolonne 109 aufgegeben wird. Da keine Absorptionskolonne vorhanden ist, wird hierfür kein Rücklauf benötigt. Ein flüssiger Stoffstrom I' wird daher nach Entspannung einer Erwärmung in den Wärmetauschern 107, 105, 103 und 101 zugeführt und aus dem Verfahren 100 ausgeführt.

Ein nicht verflüssigter Anteil des Stoffstroms O wird auch hier in Form des Stoffstroms Q aus dem Abscheidebehälter 113 abgezogen, nun jedoch zunächst in dem Wärmetauscher 105 erwärmt, anschließend durch einen weiteren Wärmetauscher 115 geführt und in einem Booster 117 verdichtet. Anschließend wird der Stoffstrom Q wieder durch die Wärmetauscher 117, 105 und 107 geführt, entspannt, mit dem Stoffstrom I' vereinigt und schließlich in den Wärmetauschern 107, 105, 103 und 101 erwärmt und aus dem Verfahren 100 ausgeführt.

Durch den erläuterten Betrieb der Rektifikationskolonne 109 kann auch hier aus deren Sumpf ein flüssiger Stoffstrom R abgezogen werden, der reich an Kohlenwasserstoffen mit zwei Kohlenstoffatomen ist. Dieser wird auch in dem Verfahren 100 beispielsweise einer Trennung zur Gewinnung vom Ethan und Ethylen in einem sogenannten C2-Splitter unterworfen.

## Patentansprüche

1. Verfahren (100) zur trenntechnischen Bearbeitung eines Ausgangsgemischs (A), das überwiegend Wasserstoff, Methan und Kohlenwasserstoffe mit zwei oder zwei und mehr Kohlenstoffatomen enthält, wobei zumindest ein Teil des Ausgangsgemischs (A) unter Bildung eines oder mehrerer Kondensate (C, F, K', M) unter Verwendung eines oder mehrerer Wärmetauscher (101, 103, 105, 107) abgekühlt und zumindest ein Teil des oder der Kondensate (C, F, K', M) unter Bildung einer gasförmigen methanreichen Fraktion einer Rektifikation unterworfen wird, **dadurch gekennzeichnet, dass** die gasförmige methanreiche Fraktion zur Bildung eines ersten Fluidstroms (Q) verwendet wird, der zumindest zum Teil in gegenüber der gasförmigen methanreichen Fraktion unveränderter Zusammensetzung auf ein Verflüssigungsdruckniveau von 35 bis 45 bar verdichtet, durch Abkühlen zumindest teilweise verflüssigt, und auf ein Abgabedruckniveau entspannt wird, und dass der erste Fluidstrom (Q) oder ein zweiter, unter Verwendung des ersten Fluidstroms (Q) gebildeter Fluistrom in dem oder zumindest einem der Wärmetauscher (101, 103, 105, 107) erwärmt wird.

2. Verfahren (100) nach Anspruch 1, bei dem die gasförmige methanreiche Fraktion auf einem Temperaturniveau von -95 bis -100 °C gebi Idet wird.

3. Verfahren (100) nach Anspruch 1 oder 2, bei dem das Abkühlen des Ausgangsgemischs (A) oder von dessen Teil in dem oder den Wärmetauschern (101, 103, 105, 107) das Übertragen von Wärme auf die verdichtete und zumindest teilweise verflüssigte methanreiche Fraktion oder deren Teil umfasst.

4. Verfahren (100) nach einem der vorstehenden Ansprüche, bei dem das Abkühlen des Ausgangsgemischs (A) oder von dessen Teil in dem oder den Wärmetauschern (101, 103, 105, 107) auf einem Abkühldruckniveau unterhalb des Verflüssigungsdruckniveaus der methanreichen Fraktion durchgeführt wird.

5. Verfahren (100) nach Anspruch 4, bei dem die Abkühlung auf einem Abkühldruckniveau von 25 bis 40 bar durchgeführt wird, und bei dem die Rektifikation auf einem Rektifikationsdruckniveau 0,2 bis 4 bar unterhalb des Abkühldruckniveaus durchgeführt wird.

6. Verfahren (100) nach Anspruch 5, bei dem eine bei dem Abkühlen des Ausgangsgemischs (A) oder von dessen Teil in dem oder den Wärmetauschern (101, 103, 105, 107) gasförmig bleibende, wasserstoffreiche Fraktion (N) ebenfalls in dem oder zumindest einem der Wärmetauscher (101, 103, 105, 107) erwärmt wird.

7. Verfahren (100) nach Anspruch 6, bei dem die wasserstoffreiche Fraktion (N) oder deren Teil auf dem Abkühldruckniveau erwärmt wird.

8. Verfahren nach einem der vorstehenden Ansprüche, bei dem mittels der Rektifikation eine flüssige, methanreiche Fraktion (I') gebildet wird, die zumindest zum Teil zusammen mit der verdichteten und zumindest teilweise verflüssigten methanreichen Fraktion oder deren Teil in dem oder den Wärmetauschern (101, 103, 105, 107) erwärmt wird.

9. Verfahren nach einem der vorstehenden Ansprüche, bei dem das Abkühlen unter Verwendung eines ersten Wärmetauschers (101), eines zweiten Wärmetauschers (103), eines dritten Wärmetauschers (105) und eines vierten Wärmetauschers (107) durchgeführt wird.

10. Verfahren nach Anspruch 9, bei dem der erste Wärmetauscher (101) unter Verwendung eines ethylenreichen Kältemittels (B) mit -50 bis -60 °C, der zweite Wärmetauscher (103) unter Verwendung eines ethylenreichen Kältemittels (E) mit -75 bis -85 °C und der dritte Wärmetauscher (105) u nter Verwendung eines ethylenreichen Kältemittels (H) mit -95 bis -105 °C betrieben wird.

11. Verfahren nach Anspruch 9 oder 10, bei dem das Ausgangsgemisch (A) oder dessen Teil nacheinander durch den ersten, den zweiten, den dritten und den vierten Wärmetauscher (101, 103, 105, 107) geführt wird, wobei stromab jedes Wärmetauschers jeweils ein Kondensat (C, F, K', M) abgeschieden wird.

12. Verfahren nach Anspruch 11, bei dem durch den vierten Wärmetauscher (107) Fraktionen einer nach dem Abkühlen in dem dritten Wärmetauscher (105) gasförmig verbliebenen und zuvor in dem vierten Wärmetauscher (107) abgekühlten Fraktion (N) erwärmt werden.

13. Verfahren nach einem der Ansprüche 9 bis 12, bei dem die methanreiche Fraktion oder deren Teil nacheinander in dem dritten Wärmetauscher (105) erwärmt, durch einen weiteren Wärmetauscher (115) geführt, auf das Verflüssigungsdruckniveau verdichtet, durch den weiteren Wärmetauscher (115) geführt und in dem dritten und vierten Wärmetauscher (105, 107) abgekühlt wird.

14. Anlage zur trenntechnischen Bearbeitung eines Ausgangsgemischs (A), das überwiegend Wasserstoff, Methan und Kohlenwasserstoffe mit zwei oder zwei und mehr Kohlenstoffatomen enthält, mit Mitteln, die dafür eingerichtet sind, zumindest ein Teil des Ausgangsgemischs unter Bildung eines oder mehrerer Kondensate (C, F, K', M) unter Verwendung eines oder mehrerer Wärmetauscher (101, 103, 105, 107) abzukühlen und zumindest ein Teil des oder der Kondensate (C, F, K', M) unter Bildung einer gasförmigen methanreichen Fraktion einer Rektifikation zu unterwerfen, **gekennzeichnet durch** Mittel, die dafür eingerichtet sind, die gasförmige methanreiche Fraktion zur Bildung eines ersten Fluidstroms (Q) zu verwenden, durch Mittel, die dafür eingerichtet sind, den ersten Fluidstrom (Q) zumindest zum Teil auf ein Verflüssigungsdruckniveau von 35 bis 40 bar zu verdichten, durch Abkühlen zumindest teilweise zu verflüssigen, und auf ein Abgabedruckniveau zu entspannen, und durch Mittel, die dafür eingerichtet sind, den ersten Fluidstrom (Q) oder einen zweiten, unter Verwendung des ersten Fluidstroms (Q) gebildeten Fluidstrom (I') in dem oder zumindest einem der Wärmetauscher (101, 103, 105, 107) zu erwärmen.

## Claims

1. Method (100) for processing a feed mixture (A) in a separation process, which predominantly contains hydrogen, methane and hydrocarbons having two or more carbon atoms, wherein at least a portion of the feed mixture (A) is cooled using one or more heat exchangers (101, 103, 105, 107) to form one or more condensates (C, F, K', M) and at least a part of the condensate or condensates (C, F, K', M) is subjected to rectification to form a gaseous methane-rich fraction, **characterized in that** the gaseous methane-rich fraction is used to form a first fluid stream (Q) which is compressed at least in part into a composition that is unchanged with respect to the gaseous methane-rich fraction to a condensing pressure level of 35 to 45 bar, liquefied at least in part by cooling, and relieved to a delivery pressure level, **and that** the first fluid stream (Q) or a second fluid stream formed using the first fluid stream (Q) is heated in the or at least one of the heat exchangers (101, 103, 105, 107).

2. Method (100) according to claim 1, wherein the gaseous methane-rich fraction is formed at a temperature level of from -95 to -100 °C.

3. Method (100) according to claim 1 or 2, wherein the cooling of the feed mixture (A) or part thereof in the heat exchanger or the heat exchangers (101, 103, 105, 107) comprises transferring heat to the compressed and at least partially liquefied methane-rich fraction or part thereof.

4. Method (100) according to any of the preceding claims, wherein the cooling of the feed mixture (A) or part thereof in the heat exchanger or the heat exchangers (101, 103, 105, 107) is carried out at a cooling pressure level below the condensing pressure level of the methane-rich fraction.

5. Method (100) according to claim 4, wherein the cooling is carried out at a cooling pressure level of 25 to 40 bar, and wherein the rectification is carried out at a rectification pressure level of 0.2 to 4 bar below the cooling pressure level.

6. Method (100) according to claim 5, wherein a hydrogen-rich fraction (N), which remains gaseous during the cooling of the feed mixture (A) or part thereof in the heat exchanger or the heat exchangers (101, 103, 105, 107), is likewise heated in the or at least one of the heat exchangers (101, 103, 105, 107).

7. Method (100) according to claim 6, wherein the hydrogen-rich fraction (N) or part thereof is heated at the cooling pressure level.

8. Method according to any one of the preceding claims, wherein the rectification forms a liquid, methane-rich fraction (I') which is at least partially heated together with the compressed and at least partially liquefied methane-rich fraction or part thereof in the heat exchanger or the heat exchangers (101, 103, 105, 107).

9. Method according to any one of the preceding claims, wherein the cooling is carried out using a first heat exchanger (101), a second heat exchanger (103), a third heat exchanger (105) and a fourth heat exchanger (107).

10. Method according to claim 9, wherein the first heat exchanger (101) is operated at -50 to -60 °C using an ethylene-rich refrigerant (B), the second heat exchanger (103) is operated at from -75 to -85 °C using an ethylene-rich refrigerant (E) and the third heat exchanger (105) is operated at -95 to -105 °C using an ethylene-rich refrigerant (H).

11. Method according to claim 9 or 10, wherein the feed mixture (A) or part thereof is passed successively through the first, the second, the third, and the fourth heat exchangers (101, 103, 105, 107), wherein a condensate (C, F, K', M) is separated in each case downstream of each heat exchanger.

12. Method according to claim 11, wherein fractions of a fraction (N) remaining gaseous after cooling in the third heat exchanger (105) and cooled beforehand in the fourth heat exchanger (107) are heated by the fourth heat exchanger (107).

13. Method according to one of claims 9 through 12, wherein the methane-rich fraction or part thereof is successively heated in the third heat exchanger (105), passed through a further heat exchanger (115), compressed to the condensing pressure level, passed through the further heat exchanger (115) and cooled in the third and fourth heat exchangers (105, 107).

14. System for processing a feed mixture (A) in a separation process, which predominantly contains hydrogen, methane and hydrocarbons having two or more carbon atoms, with means configured to cool at least a portion of the feed mixture using one or more heat exchangers (101, 103, 105, 107) to form one or more condensates (C, F, K', M) and to subject at least a part of the condensate or condensates (C, F, K', M) to rectification to form a gaseous methane-rich fraction, **characterized by** means configured to use the gaseous methane-rich fraction to form a first fluid stream (Q), **by** means configured to compress the first fluid stream (Q) at least in part to a condensing pressure level of 35 to 45 bar, **by** cooling to liquefy at least in part, and to relieve to a delivery pressure, and **by** means configured to heat the first fluid stream (Q) or a second fluid stream formed using the first fluid stream (I') in the or at least one of the heat exchangers (101, 103, 105, 107).

## Revendications

1. Procédé (100) pour le traitement par une technique de séparation d'un mélange de départ (A) qui contient principalement de l'hydrogène, du méthane et des hydrocarbures comprenant deux ou deux et plus de deux atomes de carbone, au moins une partie du mélange de départ (A) étant refroidie à l'aide d'un ou de plusieurs échangeurs thermiques (101, 103, 105, 107) avec formation d'un ou de plusieurs condensats (C, F, K', M) et au moins une partie du ou des condensats (C, F, K', M) étant soumise à une rectification avec formation d'une fraction gazeuse riche en méthane, **caractérisé en ce que** la fraction gazeuse riche en méthane est utilisée pour former un premier flux fluidique (Q), qui est comprimé au moins en partie, avec une composition non modifiée par rapport à la fraction gazeuse riche en méthane, à un niveau de pression de liquéfaction de 35 à 45 bars, liquéfié au moins partiellement par refroidissement et détendu à un niveau de pression de distribution **et en ce que** le premier flux fluidique (Q) ou un deuxième flux fluidique formé à l'aide du premier flux fluidique (Q) est réchauffé dans l'échangeur thermique ou dans au moins un des échangeurs thermiques (101, 103, 105, 107).

2. Procédé (100) selon la revendication 1, dans lequel la fraction gazeuse riche en méthane est formée à un niveau de température de -95 à -100 °C.

3. Procédé (100) selon la revendication 1 ou 2, dans lequel le refroidissement du mélange de départ (A) ou d'une partie de celui-ci dans le ou les échangeurs thermiques (101, 103, 105, 107) comprend le transfert de chaleur à la fraction riche en méthane comprimée et au moins partiellement liquéfiée ou à une partie de celle-ci.

4. Procédé (100) selon l'une quelconque des revendications précédentes, dans lequel le refroidissement du mélange de départ (A) ou d'une partie de celui-ci dans le ou les échangeurs thermiques (101, 103, 105, 107) est réalisé à un niveau de pression de refroidissement inférieur au niveau de pression de liquéfaction de la fraction riche en méthane.

5. Procédé (100) selon la revendication 4, dans lequel le refroidissement est réalisé à un niveau de pression de refroidissement de 25 à 40 bars et dans lequel la rectification est réalisée à un niveau de pression de rectification inférieur de 0,2 à 4 bars au niveau de pression de refroidissement.

6. Procédé (100) selon la revendication 5, dans lequel une fraction (N) riche en hydrogène, restant gazeuse lors du refroidissement du mélange de départ (A) ou d'une partie de celui-ci dans le ou les échangeurs thermiques (101, 103, 105, 107), est également réchauffée dans l'échangeur thermique ou dans au moins un des échangeurs thermiques (101, 103, 105, 107).

7. Procédé (100) selon la revendication 6, dans lequel la fraction (N) riche en hydrogène ou une partie de celle-ci est réchauffée au niveau de pression de refroidissement.

8. Procédé selon l'une quelconque des revendications précédentes, dans lequel une fraction (I') riche en méthane, liquide, est formée au moyen de la rectification, laquelle fraction est réchauffée au moins en partie conjointement avec la fraction riche en méthane comprimée et au moins partiellement liquéfiée ou avec une partie de celle-ci dans le ou les échangeurs thermiques (101, 103, 105, 107).

9. Procédé selon l'une quelconque des revendications précédentes, dans lequel le refroidissement est réalisé avec utilisation d'un premier échangeur thermique (101), d'un deuxième échangeur thermique (103), d'un troisième échangeur thermique (105) et d'un quatrième échangeur thermique (107).

10. Procédé selon la revendication 9, dans lequel le premier échangeur thermique (101) fonctionne, avec utilisation d'un fluide frigorigène riche en éthylène (B), de -50 jusqu'à -60 °C, le deuxième échangeur thermique (103) fonctionne, avec utilisation d'un fluide frigorigène riche en éthylène (E), de -75 jusqu'à -85 °C et le troisième échangeur thermique (105) fonctionne, avec utilisation d'un fluide frigorigène riche en éthylène (H), de -95 jusqu'à -105 °C.

11. Procédé selon la revendication 9 ou 10, dans lequel le mélange de départ (A) ou une partie de celui-ci est guidé(e) successivement à travers le premier, le deuxième, le troisième et le quatrième échangeur thermique (101, 103, 105, 107), un condensat (C, F, K', M) étant à chaque fois séparé en aval de chaque échangeur thermique.

12. Procédé selon la revendication 11, dans lequel des fractions d'une fraction (N) restant gazeuse après le refroidissement dans le troisième échangeur thermique (105) et refroidie au préalable dans le quatrième échangeur thermique (107) sont réchauffées par le quatrième échangeur thermique (107).

13. Procédé selon l'une quelconque des revendications 9 à 12, dans lequel la fraction riche en méthane ou une partie de celle-ci est successivement réchauffée dans le troisième échangeur thermique (105), guidée à travers un autre échangeur thermique (115), comprimée au niveau de pression de liquéfaction, guidée à travers l'autre échangeur thermique (115) et refroidie dans le troisième et le quatrième échangeur thermique (105, 107).

14. Installation pour le traitement par une technique de séparation d'un mélange de départ (A) qui contient principalement de l'hydrogène, du méthane et des hydrocarbures comprenant deux ou deux et plus de deux atomes de carbone, présentant des moyens conçus pour refroidir au moins une partie du mélange de départ à l'aide d'un ou de plusieurs échangeurs thermiques (101, 103, 105, 107) avec formation d'un ou de plusieurs condensats (C, F, K', M) et pour soumettre au moins une partie du ou des condensats (C, F, K', M) à une rectification avec formation d'une fraction gazeuse riche en méthane, **caractérisée par** des moyens conçus pour utiliser la fraction gazeuse riche en méthane pour former un premier flux fluidique (Q), **par** des moyens conçus pour comprimer le premier flux fluidique (Q) au moins en partie à un niveau de pression de liquéfaction de 35 à 40 bars, pour le liquéfier au moins partiellement par refroidissement et le détendre à un niveau de pression de distribution et **par** des moyens conçus pour réchauffer le premier flux fluidique (Q) ou un deuxième flux fluidique (I') formé à l'aide du premier flux fluidique (Q) dans l'échangeur thermique ou dans au moins un des échangeurs thermiques (101, 103, 105, 107).
